# EUROPEAN PATENT SPECIFICATION

(11) **EP 3 870 248 B1**
(45) Date of publication and mention of the grant of the patent: **14.10.2026**
(21) Application number: 18938154.4
(22) Date of filing: 23.10.2018
(51) Int. Cl.: A61M 1/00, A61M 3/02

(54) **FLUID HANDLING ASSEMBLY AND RELATED TUBE SET AND METHOD FOR USE IN SURGICAL PROCEDURES**
FLUIDHANDHABUNGSANORDNUNG UND ZUGEHÖRIGER SCHLAUCHSATZ UND VERFAHREN ZUR VERWENDUNG IN CHIRURGISCHEN VERFAHREN
ENSEMBLE MANIPULATION DE FLUIDE ET ENSEMBLE DE TUBES ASSOCIÉS ET PROCÉDÉ D'UTILISATION DANS DES INTERVENTIONS CHIRURGICALES

(43) Date of publication of application: 01.09.2021
(73) Proprietor: Misonix, LLC, Farmingdale, NY 11735 (US)
(72) Inventor: PAYNE, Timothy, John, Santa Ana, CA 92706 (US); CONWAY, Scott, LaVoy, Yorba Linda, CA 92886 (US); MAYERCHECK, Robert, Paul, Irvine, CA 92614 (US)
(74) Representative: Cooley (UK) LLP
(86) International application number: PCT/US2018/057070
(87) International publication number: WO 2020/086063

(56) References cited:
- WO-A1-2017/083733
- US-A- 5 403 277
- US-A- 5 460 490
- US-A1- 2008 154 095
- US-A1- 2011 300 010
- US-A1- 2012 065 482
- US-A1- 2013 131 585
- US-A1- 2016 287 779
- US-B1- 6 319 223

## Description

### BACKGROUND OF THE INVENTION

The present invention relates to moving fluids in surgical procedures. More particularly, this invention relates to a fluid handling assembly including a console and a disposable tubing set. This invention also relates to the tubing set for use with the console.

In ultrasonic surgical procedures, the head of an ultrasonically vibrating probe is placed into pressure-wave transmitting contact with tissue of a patient. The probe may be an ablating device, a debriding instrument, or a suction instrument, among others. Using an ultrasonic probe requires the delivery of an irrigant to the instrument and to the tissue in order to prevent temperature elevation to a degree that would cause necrosis. The cooling liquid must naturally be removed from the site into order to make way for further coolant. The removal entails suction that also removes entrained tissue particles.

Surgical equipment includes irrigation tubing and suction tubing as well as a pump mechanism for moving the irrigant to the surgical site, for instance, via the ultrasonic probe or a sheath surrounding the probe, and an aspiration or vacuum source connected to the suction tubing. A technician generally sets up the fluid handling circuits prior to the surgical procedure. On occasion, there are errors such as mixing the irrigation tubing and the suction tubing, which prevents proper operation of the assembly.

US2008/154095 discloses a dual pump irrigation/aspiration pump system capable of operating in a plurality of different modes suitable for a variety of different endoscopic surgical procedures. The system monitors actual or calculated intra-articular pressure and adjusts flow to maintain surgeon requested pressure at the surgical site while controlling outflow. The irrigation/aspiration pump system has an inflow pump and tubing dedicated to communicating fluid to the surgical work site and an outflow pump and tubing dedicated to removing fluid from the work site at a controlled rate. The system further has different size inflow and outflow pumps and tubing cassettes, means for altering the outflow fluid flow rate to accommodate a surgical tool and a means for declogging the surgical tool in the event of blockage. In a preferred embodiment a pressure control system provides inferred pressure information representative of the pressure at the work site.

US2016/287779 relates to a fluid management system including a disposable cassette carrying inflow and outflow tubing sections that are configured for releasably mating with a console including a control unit and a pair of roller pump heads. The console may automatically identify the disposable cassette and weigh the fluid in an inflow source. During operation, the system can monitor pressure in the working space, and provide for inflow and outflow control to maintain any desired operating parameters.

### SUMMARY OF THE INVENTION

The present invention aims to provide a tubing set and associated console that facilitate the preparation of a fluids handling system prior to surgery.

The present invention further aims to provide such a tubing set and associated console that significantly reduce, if not eliminate, errors in setting up a surgical fluids handling assembly. The present invention claims a console according to claim 1 and a tubing set according to claim 6. Preferred embodiments of the console and the tubing set are respectively claimed in dependent claims 2-5 and claims 7-11.

### Summary of the disclosure

The present disclosure describes a fluid handling system for a surgical treatment which comprises a console and a tubing set.

The console includes a housing having a panel, a peristaltic pump, a pinch valve, and a first seat and a second seat on the housing panel. The peristaltic pump is mounted to the housing and extends outwardly from the panel. More specifically, the pump includes a roller set and an anvil that are movably attached to a pump casing, the roller set being rotatable about an axis perpendicular to the housing panel and the anvil being movable from a neutral position to a closed position for clamping a tube between the roller set and a curved inside surface of the anvil. The pinch valve is mounted to the housing panel and has a slot or gap between two jaws movable relative to one another to selectively restrict flow through a tube inserted into the slot. The peristaltic pump and the pinch valve are spaced from one another along a line. The seats are disposed on the housing panel on opposite sides of the line.

The tubing set, which is intended to be disposable rather than reusable, includes an irrigation tube, an aspiration tube, a first attachment element, and a second attachment element. The irrigation tube and the aspiration tube are each coupled to both the first attachment element and the second attachment element. The first attachment element is seatable in the first seat on the console housing panel while the second attachment element is seatable in the second seat. The irrigation tube and the aspiration tube are coupled to the first attachment element and the second attachment element so that the irrigation tube and the aspiration tube extend in spaced relation to one another between the first attachment element and the second attachment element, at least when the attachment elements are spaced from one another by a distance approximately equal to the distance between the first seat and the second seat on the console panel. The irrigation tube is positionable in operative engagement with the roller set and the anvil while the aspiration tube is insertable into the slot of the pinch valve upon seating of the first attachment element in the first seat and seating of the second attachment element in the second seat.

The console preferably further comprises first coupling elements at the first seat and the second seat, while the first attachment element and the second attachment element include second coupling elements that cooperate with respective ones of the first coupling elements to releasably fasten the first attachment element and the second attachment element to the housing panel at the first seat and the second seat, respectively.

The coupling elements may include magnets. Other, alternative, coupling elements may take the form of snap-lock fasteners, adhesive layers, hook-and-loop fasteners, etc.

The seats are recesses of different geometries in the console housing panel. The first attachment element and the second attachment element have geometries corresponding to the geometries of respective ones of the recesses. Thus at least one of the attachment elements, said the second element, is disposable only in the second seat and cannot be seated in the first seat owing to a mismatch in size or shape.

The first attachment element and the second attachment element are disks of different sizes, the first seat and the second seat having different sizes congruent with respective ones of the disks. The disks may be provided on one side (an outer side) with finger grips for facilitate manipulation of the tubing set during assembly of the fluid handling system.

The console may further include a plurality of indicators such as LED lights that signal successful seating of the first attachment element and the second attachment element in the first seat and the second seat, respectively, and successful placement of the irrigation tube between the roller set and the anvil of the peristaltic pump.

The console may also include an air bubble detector at the second seat. The air bubble detector is so located to be juxtaposed to the irrigation tube upon seating of the second attachment element in the second seat. More particularly, the air bubble detector may be housed in a projection on the second seat that is inserted into a hole in the rear or inner side of the second attachment element. The irrigation tube extends across the hole so as to automatically insert into a slot in the projection upon the seating of the second attachment element in the second seat on the housing panel of the console. The air bubble detector may take the form of an ultrasound sensor.

The panel is preferably a side panel of the housing, and the peristaltic pump and the pinch valve are preferably located vertically one above the other.

The present disclosure is directed in part to a fluid handling tubing set for use in surgical interventions. The tubing set, described above, is utilizable with a console as described above.

Concomitantly, the present disclosure contemplates a console as described above.

A surgical method in accordance with the present disclosure comprises providing a tubing set for carrying fluids, the tubing set including a first tube, a second tube, a first attachment element, and a second attachment element, the first tube and the second tube each being coupled to both the first attachment element and the second attachment element. The first attachment element and the second attachment element have different geometries. The method includes seating the first attachment element in a first seat on a panel of a console and seating the second attachment element in a second seat on the panel of the console so that the first tube and the second tube extend in spaced relation to one another between the first attachment element and the second attachment element. Thereafter, the first tube is manipulated so as to dispose a portion of the first tube in operative engagement with a roller set and an anvil of the console projecting from the panel, and the second tube is manipulated so as to insert the second tube into a slot or gap of a pinch valve of the console projecting from the panel.

### BRIEF DESCRIPTION OF THE DRAWINGS

FIG. 1 is a perspective view of a fluid handling system particularly including a tubing set and a console, showing the tubing set spaced from and in position for attachment to the console, in accordance with the present invention.
FIG. 2 is a perspective view of the system of FIG. 1, showing the tubing system positioned on the console, with an irrigation tube between an anvil and a roller set of a peristaltic pump.
FIG. 3 is a perspective view similar to FIG. 2, showing a cover of the peristaltic pump closed over the irrigation tube and further showing an aspiration tube inserted between jaws (or an anvil and jaw) of a pinch solenoid on the console.

### DETAILED DESCRIPTION

A fluid handling system 10 for use in a surgical treatment procedure includes a console 12 and a tubing set 14.

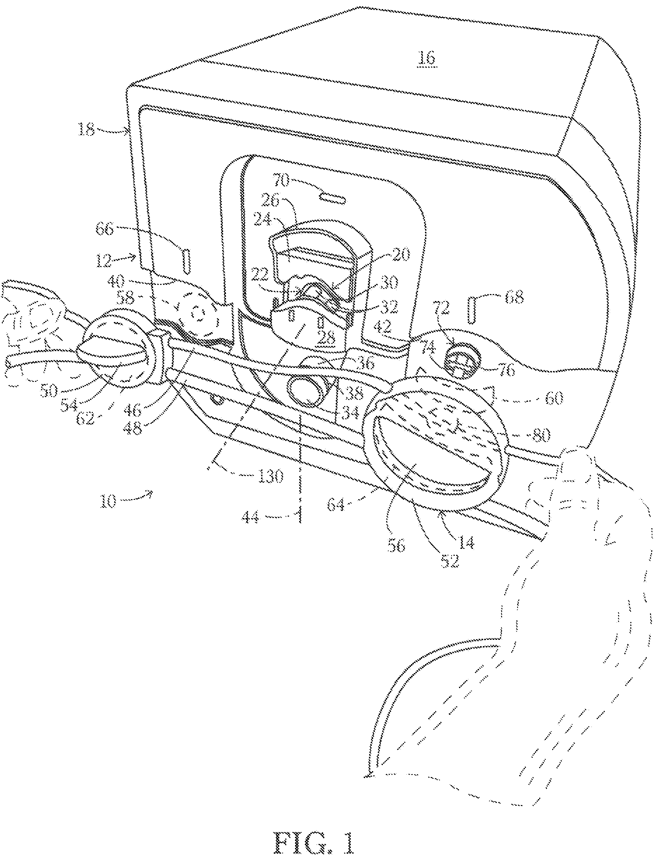

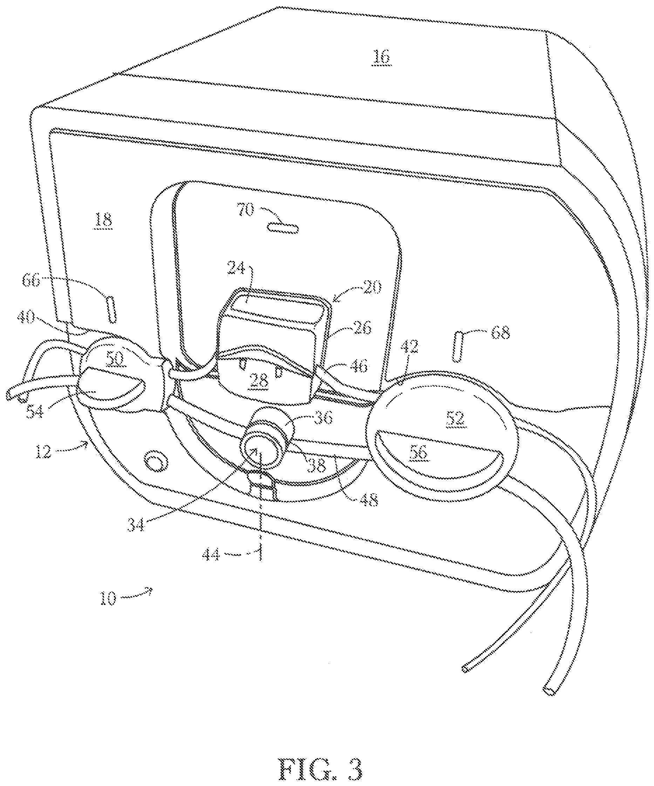

Console 12 includes a housing 16 with a side panel 18. A peristaltic pump 20 is mounted to housing 16 on side panel 18 and includes a roller set 22 and an anvil 24, as well as a cover 26. Roller set 22 is held at an outer end by a bracket 28 for rotation about an axis 130 oriented perpendicular to panel 18, Cover 26 is hingedly mounted to a pump casing (not separately designated) and, when rotated downwardly from a neutral position shown in FIG. 1 to a closed position shown in FIG. 3, moves anvil 24 toward roller set 22, so that a curved surface or face 30 of the anvil is placed adjacent to rollers 32 of roller set 22.

Console 12 further includes a solenoid-operated pinch valve 34 that comprises two relatively movable jaws (not separately designated) disposed inside a sleeve 36 mounted to panel 18 and formed with a transverse slot 38.

Console 12 further includes, molded into side panel 18, a first seat 40 and a second seat 42 in the form of circular or oval recesses of different geometries. More specifically, seats 40 and 42 may be of different shapes and/or different sizes. In the embodiment illustrated in the drawings, seat 42 is larger than seat 40.

Peristaltic pump 20 and solenoid pinch valve 34 are disposed one above the other and thereby define a vertical line or plane 44. Seats 40 and 42 are disposed on side panel 18 on opposite sides of line or plane 44.

Tubing set 14 includes an irrigation tube 46, an aspiration tube 48, a first attachment disk 50, and a second attachment disk 52. Irrigation tube 46 and aspiration tube 48 are each coupled to both first attachment disk 50 and second attachment disk 52. Disks 50 and 52 may be formed by bonding two half disks to one another. sandwiching tubes 46 and 48 between the half disks in each case. Disks 50 and 52 are each provided on an outer side or face (not designated) with a respective finger grip 54 and 56 in the form of a ring or flange the facilitating manipulation of the disks during an assembly procedure.

Typically, a technician grasping finger grips 54 and 56 will place disks 50 and 52 in recesses or seats 40 and 42. Attachment is of a quick-release type owing to magnets 58 and 60 (or magnets and metal elements) positioned in housing 16 adjacent seats 40 and 42 and magnetic counterparts 62 and 64 attached to disks 50 and 52. Alternative coupling elements such as snap-lock fasteners, adhesive layers, hook-and-loop fasteners, etc., may be used instead of magnets.

Disks 50 and 52 have different geometries, that is, different sizes and/or shapes, that are respectively congruent with the geometries of seats 40 and 42 so that disks 50 and 52 are seatable only in seats 40 and 42, respectively. This ensures that irrigation tube 46 is located above aspiration tube 48 once attachment disks 50 and 52 are seated on panel 18. Irrigation tube 46 and aspiration tube 48 are coupled to attachment disks 50 and 52 so that tubes 46 and 48 extend in spaced relation to one another between the disks, when the disks are placed into seats 40 and 42.

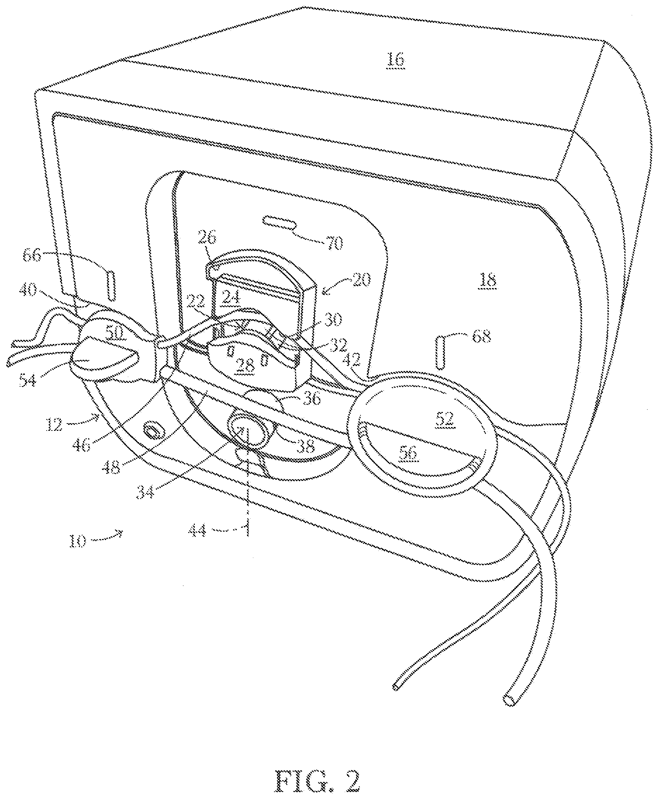

Disks 50 and 52 are spaced from one another by lengths of tubes 46 and 48 that are approximately equal (and slightly greater) to the distance between seats 40 and 42. Disks 50 and 52 are held and manipulated via flanges or finger grips 54 and 56, to engage housing side panel 18 in recesses or seats 40 and 42. Upon a disposition of disks 50 and 52 in seats 40 and 42, irrigation tube 46 is placed over roller set 22 in a gap between anvil 24 and bracket 28, as shown in FIG. 2. One then rotates the cover 26, which is pivotably mounted to housing 16 via anvil 24, thereby moving the anvil towards roller set 22 and clamping tube 46 between anvil surface 30 and the rollers 32, as illustrated in FIG. 3. As further shown in FIG. 3, aspiration tube 48 is inserted into slot 38 of pinch valve 34.

Console 12 exhibits indicators lights 66, 68, 70 that turn from one color, such as red or blue to another color such as green upon a successful seating of disks 50 and 52 in seats 40 and 42 and a successful or effective disposition of tube 46 between pump set 22 and surface 30 of anvil 24.

Console 12 may also include an air bubble detector 72 within seat 42. Bubble detector 72 preferably takes the form of an ultrasonic sensor with transmitter and receiver elements (not separately depicted) disposed in respective halves 74 and 76 of a slotted projection. During a seating of attachment or coupling disk 52 in recess or seat 42, the detector 72 is inserted into a hole 80 in the rear or inner side of attachment disk 52. Irrigation tube 46 extends across the hole 80 so as to automatically insert into a slot (not designated) defined between receiver halves 74 and 76 in the detector 72 upon the seating of attachment disk 52 in seat 42.

Panel 18 is preferably a side panel of housing 16, and peristaltic pump 20 and pinch valve 34 are preferably located vertically one above the other. However different configurations are possible. The essential feature is that the shapes and or sizes of disks 50 and 52, as well as seats 40 and 42, preclude an incorrect positioning of tubes 46 and 48. Upon the placements of disks 50 and 52 in recesses 40 and 42, it is not possible to position aspiration tube 48 on roller set 22 in the gap between anvil 24 and bracket or bearing plate 28. Only irrigation tube 46 is positionable there.

Prior to an ultrasonic surgical procedure, wherein liquid irrigant or coolant is furnished to an ultrasonic instrument via tube 46 and removed from the surgical site via tube 48, tubing set 14 is manipulated as described above so as to dispose a portion of tube 46 in operative engagement with roller set 22 and anvil 24, and a portion of tube 48 is inserted into slot or gap 38 of pinch valve 34.

Pursuant to the present invention, tubing set 14 includes irrigant tube 46 and suction or aspiration tube 48 connected in parallel to one another via a pair of spaced fasteners, that is, disks 50 and 52. Fasteners or attachment disks 50 and 52 include magnetic elements and have with planar projections 54 and 56 that serve as grips for grasping between the forefingers and thumbs by a technician. Disks 50 and 52 are juxtaposed to delineated positions (recesses or seats 40 and 42) on side panel 18 of console 16 where the disks are secured to the console in a quick-release mode of attachment, owing to magnetic attraction. Console panel 18 features well-known prior-art peristaltic pump 20 and a well-known pinch solenoid 34 that are spaced from one another. Disks 50 and 52 are attached to console 16 on opposite sides of an imaginary line 44 or plane extending defined by the locations of peristaltic pump 22 and pinch solenoid 34, preferably one located above the other. After the magnetic attachment, irrigation tube 46 is inserted over the rollers 32 of pump 20 and a cover- or lid-type anvil 24 is pivoted into place so that irrigation tube 46 is pressed between the pump rollers 32 and a curved inner surface 30 of the anvil. Aspiration tube 48 is placed in a gap 38 defined between jaws (not visible owing to sleeve 36) of pinch solenoid 34. Tubing set 14 simplifies the process of tube placement, making it impossible to insert the aspiration tube 48 into peristaltic pump 20.

Disk 50 or 52 may be provided with an RFID tag or chip (not shown) that may be set by a transmitter in housing 16 to prevent re-use of the tubing set 14, primarily for ensuring sterility.

Although the invention has been described in terms of particular embodiments and applications, one of ordinary skill in the art, in light of this teaching, can generate additional embodiments and modifications without exceeding the scope of the claimed invention. Moreover, the phase shift might be varying, for instance, where the vibration modes are of different frequencies. Accordingly, it is to be understood that the drawings and descriptions herein are proffered by way of example to facilitate comprehension of the invention and should not be construed to limit the scope thereof.

## Claims

1. A console (12) for a surgical treatment utilizable with a tubing set (14) including:
an irrigation tube (46);
an aspiration tube (48);
a first attachment element (50); and
a second attachment element (52), wherein said irrigation tube (46) and said aspiration tube (48) are each coupled to both said first attachment element (50) and said second attachment element (52);
said console (12) including:
a housing (16) having a panel (18);
a peristaltic pump (20) mounted to said housing (16) and extending outwardly from said panel (18), said pump (20) including a roller set (22) and an anvil (24) each movable relative to said panel (18);
a pinch valve (34) mounted to said housing (16), said pinch valve (34) having a slot (38) or gap between two jaws movable relative to one another, said peristaltic pump (20) and said pinch valve (34) being spaced along a line from one another; and
a first seat (40) and a second seat (42) on said panel (18) disposed on opposite sides of the line, wherein said first attachment element (50) is seatable in said first seat (40) and said second attachment element (52) is seatable in said second seat (42),
said irrigation tube (46) being disposable in operative engagement with said roller set (22) and said anvil (24) and said aspiration tube (48) being insertable into said slot (38) or gap upon seating of said first attachment element (50) in said first seat (40) and seating of said second attachment element (52) in said second seat (42),
the console (12), when utilized with the tubing set (14), permits said irrigation tube (46) and said aspiration tube (48) to extend in spaced relation to one another between said first attachment element (50) and said second attachment element (52) upon seating of said first attachment element (50) in said first seat (40) and seating of said second attachment element (52) in said second seat (42).

2. The console defined in claim 1, wherein said console (12) further comprises first coupling elements (58, 60) at said first seat (40) and said second seat (42) and wherein said first attachment element (50) and said second attachment element (52) include second coupling elements (62, 64) that cooperate with respective ones of said first coupling elements (58, 60) to releasably fasten said first attachment element (50) and said second attachment element (52) to said panel (18) at said first seat (40) and said second seat (42), respectively.

3. The console defined in claim 2, wherein , said first seat (40) and said second seat (42) have different sizes in order to be congruent with a disk of said first attachment element (50) and a disk of said second attachment element (52), respectively, wherein said disks have different sizes.

4. The console defined in claim 2, wherein said first coupling elements (58, 60) are magnets and said second coupling elements (62, 64) are magnetic counterparts.

5. The console defined in claim 1, wherein said seats (40, 42) are recesses in said panel (18), each seat (40, 42) having a geometry intended to correspond to a respective geometry of one of said first attachment element (50) and said second attachment element (52).

6. A tubing set (14) for surgical interventions, said tubing set (14) utilizable with a console (12) according to claim 1,
said tubing set (14) including:
an irrigation tube (46);
an aspiration tube (48);
a first attachment element (50) seatable in said first seat (40); and
a second attachment element (52) seatable in said second seat (42),
said irrigation tube (46) and said aspiration tube (48) are each coupled to both said first attachment element (50) and said second attachment element (52) so that said irrigation tube (46) and said aspiration tube (48) extend in spaced relation to one another between said first attachment element (50) and said second attachment element (52),
said irrigation tube (46) being disposable in operative engagement with said roller set (22) and said anvil (24) and said aspiration tube (48) being insertable into said slot (38) or gap upon seating of said first attachment element (50) in said first seat (40) and seating of said second attachment element (52) in said second seat (42).

7. The tubing set (14) defined in claim 6, wherein said first attachment element (50) and said second attachment element (52) include coupling elements (62, 64) that cooperate with respective coupling elements (58, 60) on said console (12) to releasably fasten said first attachment element (50) and said second attachment element (52) to said panel (18) at said first seat (40) and said second seat (42), respectively.

8. The tubing set (14) defined in claim 7, wherein said coupling elements (58, 60, 62, 64) include magnets.

9. The tubing set (14) defined in claim 6, wherein said first attachment element (50) and said second attachment element (52) are disks of different sizes.

10. The tubing set (14) defined in claim 6, wherein said first attachment element (50) and said second attachment element (52) have different geometries.

11. The tubing set (14) defined in claim 9, wherein said disks (50, 52) are provided on one side with finger grips (54, 56).

## Patentansprüche

1. Konsole (12) für eine chirurgische Behandlung, verwendbar mit einem Schlauchsatz (14) aufweisend:
einen Spülschlauch (46);
einen Ansaugschlauch (48);
ein erstes Befestigungselement (50); und
ein zweites Befestigungselement (52), wobei der Spülschlauch (46) und der Ansaugschlauch (48) jeweils sowohl an das erste Befestigungselement (50) als auch das zweite Befestigungselement (52) gekoppelt sind;
wobei die Konsole (12) aufweist:
ein Gehäuse (16) mit einer Frontplatte (18);
eine Peristaltikpumpe (20), die an dem Gehäuse (16) montiert ist und von der Frontplatte (18) nach außen vorsteht, wobei die Pumpe (20) einen Rollensatz (22) und einen Amboss (24) umfasst, die jeweils relativ zu der Frontplatte (18) beweglich sind;
ein Quetschventil (34), das an dem Gehäuse (16) montiert ist, wobei das Quetschventil (34) einen Schlitz (38) oder Spalt zwischen zwei relativ zueinander beweglichen Backen aufweist, wobei die Peristaltikpumpe (20) und das Quetschventil (34) entlang einer Linie voneinander beabstandet sind; und
einen ersten Sitz (40) und einen zweiten Sitz (42) an der Frontplatte (18), die an gegenüberliegenden Seiten der Linie angeordnet sind, wobei das erste Befestigungselement (50) in den ersten Sitz (40) einsetzbar ist und das zweite Befestigungselement (52) in den zweiten Sitz (42) einsetzbar ist,
wobei der Spülschlauch (46) in Wirkeingriff mit dem Rollensatz (22) und dem Amboss (24) angeordnet werden kann und bei Einsetzen des ersten Befestigungselements (50) in den ersten Sitz (40) und Einsetzen des zweiten Befestigungselements (52) in den zweiten Sitz (42) der Ansaugschlauch (48) in den Schlitz (38) oder Spalt einsetzbar ist,
wobei die Konsole (12) bei Verwendung mit dem Schlauchsatz (14) ermöglicht, dass der Spülschlauch (46) und der Ansaugschlauch (48) in voneinander beabstandeter Beziehung zwischen dem ersten Befestigungselement (50) und dem zweiten Befestigungselement (52) verlaufen, wenn das erste Befestigungselement (50) in dem ersten Sitz (40) sitzt und das zweite Befestigungselement (52) in dem zweiten Sitz (42) sitzt.

2. Konsole nach Anspruch 1, wobei die Konsole (12) ferner erste Kopplungselemente (58, 60) an dem ersten Sitz (40) und dem zweiten Sitz (42) umfasst und wobei das erste Befestigungselement (50) und das zweite Befestigungselement (52) zweite Kopplungselemente (62, 64) umfassen, die mit entsprechenden der ersten Kopplungselemente (58, 60) zusammenwirken, um das erste Befestigungselement (50) und das zweite Befestigungselement (52) lösbar an dem ersten Sitz (40) bzw. dem zweiten Sitz (42) an der Frontplatte (18) zu befestigen.

3. Konsole nach Anspruch 2, wobei der erste Sitz (40) und der zweite Sitz (42) unterschiedliche Größen aufweisen, um mit einer Scheibe des ersten Befestigungselements (50) bzw. einer Scheibe des zweiten Befestigungselements (52) kongruent zu sein, wobei die Scheiben unterschiedliche Größen aufweisen.

4. Konsole nach Anspruch 2, wobei die ersten Kopplungselemente (58, 60) Magnete sind und die zweiten Kopplungselemente (62, 64) magnetische Gegenstücke sind.

5. Konsole nach Anspruch 1, wobei die Sitze (40, 42) Aussparungen in der Frontplatte (18) sind, wobei jeder Sitz (40, 42) eine Geometrie aufweist, die dazu bestimmt ist, einer entsprechenden Geometrie von einem von dem ersten Befestigungselement (50) und dem zweiten Befestigungselement (52) zu entsprechen.

6. Schlauchsatz (14) für chirurgische Eingriffe, wobei der Schlauchsatz (14) mit einer Konsole (12) nach Anspruch 1 verwendbar ist,
wobei der Schlauchsatz (14) aufweist:
einen Spülschlauch (46);
einen Ansaugschlauch (48);
ein erstes Befestigungselement (50), das in den ersten Sitz (40) einsetzbar ist; und
ein zweites Befestigungselement (52), das in den zweiten Sitz (42) einsetzbar ist,
wobei der Spülschlauch (46) und der Ansaugschlauch (48) jeweils sowohl an das erste Befestigungselement (50) als auch an das zweite Befestigungselement (52) gekoppelt sind, so dass der Spülschlauch (46) und der Ansaugschlauch (48) in voneinander beabstandeter Beziehung zwischen dem ersten Befestigungselement (50) und dem zweiten Befestigungselement (52) verlaufen,
wobei der Spülschlauch (46) in Wirkeingriff mit dem Rollensatz (22) und dem Amboss (24) angeordnet werden kann und bei Einsetzen des ersten Befestigungselements (50) in den ersten Sitz (40) und Einsetzen des zweiten Befestigungselements (52) in den zweiten Sitz (42) der Ansaugschlauch (48) in den Schlitz (38) oder Spalt einsetzbar ist.

7. Schlauchsatz (14) nach Anspruch 6, wobei das erste Befestigungselement (50) und das zweite Befestigungselement (52) Kopplungselemente (62, 64) umfassen, die mit entsprechenden Kopplungselementen (58, 60) an der Konsole (12) zusammenwirken, um das erste Befestigungselement (50) und das zweite Befestigungselement (52) an dem ersten Sitz (40) bzw. dem zweiten Sitz (42) lösbar an der Frontplatte (18) zu befestigen.

8. Schlauchsatz (14) nach Anspruch 7, wobei die Kopplungselemente (58, 60, 62, 64) Magnete aufweisen.

9. Schlauchsatz (14) nach Anspruch 6, wobei das erste Befestigungselement (50) und das zweite Befestigungselement (52) Scheiben mit unterschiedlicher Größe sind.

10. Schlauchsatz (14) nach Anspruch 6, wobei das erste Befestigungselement (50) und das zweite Befestigungselement (52) unterschiedliche Geometrien aufweisen.

11. Schlauchsatz (14) nach Anspruch 9, wobei die Scheiben (50, 52) an einer Seite mit Fingergriffen (54, 56) versehen sind.

## Revendications

1. Console (12) pour traitement chirurgical pouvant être utilisée avec un ensemble de tubes (14) comprenant :
un tube d'irrigation (46) ;
un tube d'aspiration (48) ;
un premier élément de fixation (50) ; et
un second élément de fixation (52), ledit tube d'irrigation (46) et ledit tube d'aspiration (48) étant chacun accouplés à la fois audit premier élément de fixation (50) et audit second élément de fixation (52) ;
ladite console (12) comprenant :
un boîtier (16) comprenant un panneau (18) ;
une pompe péristaltique (20) montée sur ledit boîtier (16) et s'étendant vers l'extérieur à partir dudit panneau (18), ladite pompe (20) comprenant un ensemble de rouleaux (22) et une enclume (24), chacun mobile par rapport audit panneau (18) ;
un robinet-vanne à manchon déformable (34) monté sur ledit boîtier (16), ledit robinet-vanne à manchon déformable (34) présentant une fente (38) ou un espace entre deux mâchoires mobiles l'une par rapport à l'autre, ladite pompe péristaltique (20) et ledit robinet-vanne à manchon déformable (34) étant espacés l'un de l'autre le long d'une ligne ; et
un premier siège (40) et un second siège (42) sur ledit panneau (18) disposés sur des côtés opposés de la ligne, ledit premier élément de fixation (50) pouvant être mis en place dans ledit premier siège (40) et ledit second élément de fixation (52) pouvant être mis en place dans ledit second siège (42),
ledit tube d'irrigation (46) pouvant être mis en prise fonctionnelle avec ledit ensemble de rouleaux (22) et ladite enclume (24), et ledit tube d'aspiration (48) pouvant être inséré dans ladite fente (38) ou ledit espace lors de la mise en place dudit premier élément de fixation (50) dans ledit premier siège (40) et de la mise en place dudit second élément de fixation (52) dans ledit second siège (42),
la console (12), lorsqu'elle est utilisée avec l'ensemble de tubes (14), permettant audit tube d'irrigation (46) et audit tube d'aspiration (48) de s'étendre en relation espacée l'un par rapport à l'autre entre ledit premier élément de fixation (50) et ledit second élément de fixation (52) lorsque ledit premier élément de fixation (50) est mis en place dans ledit premier siège (40) et ledit second élément de fixation (52) est mis en place dans ledit second siège (42).

2. Console selon la revendication 1, ladite console (12) comprenant en outre des premiers éléments d'accouplement (58, 60) au niveau dudit premier siège (40) et dudit second siège (42) et ledit premier élément de fixation (50) et ledit second élément de fixation (52) comprenant des seconds éléments d'accouplement (62, 64) qui coopèrent avec des éléments respectifs desdits premiers éléments d'accouplement (58, 60) pour fixer de manière libérable ledit premier élément de fixation (50) et ledit second élément de fixation (52) audit panneau (18) au niveau dudit premier siège (40) et dudit second siège (42), respectivement.

3. Console selon la revendication 2, ledit premier siège (40) et ledit second siège (42) ayant des tailles différentes afin d'être en coïncidence avec un disque dudit premier élément de fixation (50) et un disque dudit second élément de fixation (52), respectivement, lesdits disques ayant des tailles différentes.

4. Console selon la revendication 2, lesdits premiers éléments d'accouplement (58, 60) étant des aimants et lesdits seconds éléments d'accouplement (62, 64) étant des contreparties magnétiques.

5. Console selon la revendication 1, lesdits sièges (40, 42) étant des évidements dans ledit panneau (18), chaque siège (40, 42) ayant une géométrie destinée à correspondre à une géométrie respective de l'un parmi ledit premier élément de fixation (50) et ledit second élément de fixation (52).

6. Ensemble de tubes (14) pour interventions chirurgicales, ledit ensemble de tubes (14) pouvant être utilisé avec une console (12) selon la revendication 1, ledit ensemble de tubes (14) comprenant :
un tube d'irrigation (46) ;
un tube d'aspiration (48) ;
un premier élément de fixation (50) pouvant être mis en place dans ledit premier siège (40) ; et
un second élément de fixation (52) pouvant être mis en place dans ledit second siège (42),
ledit tube d'irrigation (46) et ledit tube d'aspiration (48) étant chacun accouplés à la fois audit premier élément de fixation (50) et audit second élément de fixation (52) de sorte que ledit tube d'irrigation (46) et ledit tube d'aspiration (48) s'étendent de manière espacée l'un par rapport à l'autre entre ledit premier élément de fixation (50) et ledit second élément de fixation (52),
ledit tube d'irrigation (46) pouvant être mis en prise fonctionnelle avec ledit ensemble de rouleaux (22) et ladite enclume (24), et ledit tube d'aspiration (48) pouvant être inséré dans ladite fente (38) ou ledit espace lors de la mise en place dudit premier élément de fixation (50) dans ledit premier siège (40) et de la mise en place dudit second élément de fixation (52) dans ledit second siège (42).

7. Ensemble de tubes (14) selon la revendication 6, ledit premier élément de fixation (50) et ledit second élément de fixation (52) comprenant des éléments d'accouplement (62, 64) qui coopèrent avec des éléments d'accouplement (58, 60) respectifs sur ladite console (12) pour fixer de manière libérable ledit premier élément de fixation (50) et ledit second élément de fixation (52) audit panneau (18) au niveau dudit premier siège (40) et dudit second siège (42), respectivement.

8. Ensemble de tubes (14) selon la revendication 7, lesdits éléments d'accouplement (58, 60, 62, 64) comprenant des aimants.

9. Ensemble de tubes (14) selon la revendication 6, ledit premier élément de fixation (50) et ledit second élément de fixation (52) ayant des disques de tailles différentes.

10. Ensemble de tubes (14) selon la revendication 6, ledit premier élément de fixation (50) et ledit second élément de fixation (52) ayant des géométries différentes.

11. Ensemble de tubes (14) selon la revendication 9, lesdits disques (50, 52) étant munis sur un côté de brides pour doigts (54, 56).
